(19) Europäisches Patentamt / European Patent Office / Office européen des brevets

(11) **EP 2 191 821 A1**

(12) **EUROPEAN PATENT APPLICATION**

(43) Date of publication:
02.06.2010 Bulletin 2010/22

(51) Int Cl.:
*A61K 9/14* (2006.01)     *A61K 9/72* (2006.01)
*A61K 31/4704* (2006.01)     *A61K 47/26* (2006.01)

(21) Application number: 08169993.6

(22) Date of filing: 26.11.2008

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **CHIESI FARMACEUTICI S.p.A.**
**43100 Parma (IT)**

(72) Inventors:
• **Buttini, Francesca**
**43100, PARMA (IT)**

• **Colombo, Paolo**
**43100, PARMA (IT)**
• **Parlati, Chiara**
**43100, PARMA (IT)**
• **Cocconi, Daniela**
**43100, PARMA (IT)**
• **Musa, Rossella**
**43100, PARMA (IT)**

(74) Representative: **Minoja, Fabrizio**
**Bianchetti Bracco Minoja S.r.l.**
**Via Plinio 63**
**20129 Milano (IT)**

(54) **Microparticles comprising a salt of 8-hydroxy-2-[[(1R)-2-(4-methoxyphenyl)-1-methylethyl] amino]ethyl]-2(1H)-quinolinone having improved adhesion properties for dry powder inhalation**

(57) The invention relates to powder particles comprising a pharmaceutically acceptable salt of 8-hydroxy-5-[(1R)-1-hydroxy-2-[[(1R)-2-(4-methoxyphenyl)-1-methylethyl]amino]ethyl]-2(1H)-quinolinone (carmoterol) having improved adhesion properties for dry powder formulations for inhalation.

The invention also relates to methods for preparing them, corresponding formulations in the form of powders for inhalation and uses thereof.

EP 2 191 821 A1

**Description**

**FIELD OF INVENTION**

The present invention relates to powder particles comprising a pharmaceutically acceptable salt of 8-hydroxy-5-[(1R)-1-hydroxy-2-[[(1R)-2-(4-methoxyphenyl)-1-methylethyl]amino]ethyl]-2(1H)-quinolinone (carmoterol) having improved adhesion properties for dry powder formulations for inhalation.

The invention also relates to methods for preparing them, corresponding formulations in the form of powders for inhalation and uses thereof.

**BACKGROUND OF THE INVENTION**

The administration of pharmacologically active ingredients by inhalation to the airways is a widely used technique especially for the treatment of reversible airway obstruction, inflammation and hyper-responsiveness.

Some of the most widely used systems for the administration of drugs to the airways are the dry powder inhalers (DPIs).

Drugs intended for inhalation as dry powders by means of DPIs should be used in the form of particles of few micron (mm) particle size, generally comprised from 0.5 to 10 micron. Micronisation is generally achieved by conventional milling processes.

Although micronisation of the drug is essential for deposition into the lower respiratory tract during inhalation, it is known that the finer the particles are, the stronger are the cohesion forces that favour the formation of agglomerates.

For this reason, powders for inhalation are commonly formulated by diluting the micronised drug in a pharmacologically inert physiologically acceptable excipient consisting of coarser particles to yield the so-called "interactive ordered mixtures".

However the drugs commonly utilised in powders for inhalation such as long acting beta2-agonists (hereinafter LABAs) are administered at low doses, and hence are present in the formulation in a low concentration.

Therefore, it is important to ensure that drug disperses well and is uniformly distributed in the ordered mixture as its lack of homogeneity, upon administration through the inhaler, could involve a risk of an over- or under-dosage, and hence is detrimental to the possibility of achieving a reproducible accuracy of the metered dose.

Ensuring a good homogeneity is particularly critical for very low-dosage strength drugs such as 8-hydroxy-5-[(1R)-1-hydroxy-2-[[(1R)-2-(4-methoxyphenyl)-1-methylethyl]amino]ethyl]-2(1H)-quinolinone (hereinafter indicated as carmoterol), a LABA currently under development for the treatment of respiratory diseases that should be administered at a therapeutical dose ranging from 1 to 4 $\mu$g per actuation of the inhaler.

The present inventors indeed found that agglomerates are present even though said drug in form of micronised particles is diluted by mixing with coarse excipient particles.

The presence of the agglomerates is an index of poor dispersion of the drug and jeopardizes the possibility of obtaining a uniform distribution of carmoterol in the powder mixture.

Poor dispersion, together with other properties such as high adhesiveness degree, leads to problems in the manufacturing of inhalable carmoterol-based powder compositions having good dosage reproducibility when administered by DPI's.

In the prior art, attempts to overcome this problem have been reported.

For example, WO2005089717 teaches to avoid agglomeration by preparing crystalline microparticles consisting of carmoterol and excipient particles with a defined particle size that are obtained by mechanically co-mixing or co-milling the constituents.

However the preparation of said microparticles is a time-consuming step.

WO2008084312 provides particles of the drug obtained by conventional milling processes having a well defined and narrow particle size distribution. However, the conditions for obtaining said feature are hard to reproduce from a batch size to another one, rendering the approach not very appealing for an industrial scale.

In view of the outlined problem and the limits and drawbacks of the solutions of the prior art, it would hence be highly desirable to provide powder particles comprising carmoterol as active ingredient demonstrating improved dispersibility when diluted with coarse excipient particles and at the same time good redispersion properties from the surface of the carrier during inhalation in order to give rise to a good respirable fraction.

The problem is solved by the powder particles of the present invention.

**SUMMARY OF THE INVENTION**

The present invention provides powder particles comprising a pharmaceutically acceptable salt of 8-hydroxy-5-[(1R)-1-hydroxy-2-[[(1R)-2-(4-methoxyphenyl)-1-methylethyl]amino]ethyl]-2(1H)-quinolinone (carmoterol) said particles having improved adhesion properties.

In particular it is an object of the invention to provide powder particles comprising a pharmaceutically acceptable salt of carmoterol characterized by a shape factor SF comprised between 0.8 and 1.0 and by the fact that the salt of carmoterol in said powder particles is present in a substantial amorphous form.

Advantageously, said powder particles are characterized by a force of adhesion $F_{max}$ comprised between 12 and 30 nN, preferably between 15 and 28 nN, even more preferably between 18 and 25 nN.

Optionally, the powder particles of the invention may comprise a further active ingredient and/or a pharmaceutically acceptable excipient.

The invention is also directed to methods for preparing the powder particles of the invention. In particular, according to a preferred embodiment, the powder particles of the invention are prepared by spray-drying.

In another aspect, the present invention is directed to a pharmaceutical formulation in the form of inhalable dry powder comprising the aforementioned powder particles, and coarse particles of a physiologically acceptable pharmacologically-inert solid carrier.

According to a further aspect, the present invention provides a dry powder inhaler comprising the aforementioned pharmaceutical formulation.

The powder particles of the invention are preferably administered by inhalation for the prevention and/or treatment of an inflammatory or obstructive airways disease such as asthma or chronic obstructive pulmonary disease (COPD).

Accordingly, in a further aspect, the present invention comprises the use of the powder particles described before, for the prevention and/or treatment of an inflammatory or obstructive airways disease such as asthma or chronic obstructive pulmonary disease (COPD).

In a still further aspect, the present invention comprises a method of preventing and/or treating an inflammatory and/or obstructive airways disease such as asthma or chronic obstructive pulmonary disease (COPD), which comprises administration by inhalation of an effective amount of the powder particles described before.

At last, the present invention is directed to powder particles for administration through a dry powder inhaler comprising a pharmaceutically acceptable salt of 8-hydroxy-5-[(1R)-1-hydroxy-2-[[(1R)-2-(4-methoxyphenyl)-1-methylethyl]amino]ethyl-2(1H)-quinolinone (carmoterol), obtainable by a process which comprises the following steps:

    i) dissolving the salt of carmoterol in an aqueous solution,
    ii) feeding the resulting aqueous solution into the drying chamber of a spray-drier through an atomizing device to form droplets;
    iii) introducing a stream of pre-heated drying gas into the drying chamber to form powder particles;
    iv) further drying and separating the powder particles from the moist gas.

## DEFINITIONS

Along the text, the terms "drug", "active ingredient", "active agent" and "active substance" are used as synonyms.

With the term very low-dosage strength, we refer to active ingredients endowed with particularly high potency which are present in the powder formulation in a very low concentration. Said active ingredients are commonly administered by a dry powder inhaler (DPI) at a single dose lower than 6 μg.

By "therapeutical dose" it is meant the quantity of active ingredient administered at one time by inhalation upon actuation of the inhaler. Said single dose may be delivered in one or more actuations (shots), of the inhaler. For "actuation" it is meant the release of active ingredient from the device by a single activation (e.g. mechanical or breath).

In general terms, the particle size of particles is quantified by measuring a characteristic equivalent sphere diameter, known as volume diameter, by laser diffraction.

The particle size may also be quantified by measuring the mass diameter by means of suitable instrument well known to the skilled person such as, for instance the sieve analyser.

The volume diameter (VD) is related to the mass diameter (MD) by the density of the particles (assuming a size independent density for the particles).

In the present application, the particle size is expressed in terms of mass diameter and the particle size distribution is expressed in terms of: i) the mass median diameter (MMD) which corresponds to the diameter of 50 percent by weight or volume respectively, of the particles, and ii) the MD in micron of 10% and 90% of the particles, respectively.

Upon aerosolisation, the particle size is expressed as mass aerodynamic diameter (MAD) and the particle size distribution as mass median aerodynamic diameter (MMAD). The MAD indicates the capability of the particles of being transported suspended in an air stream. The MMAD corresponds to the mass aerodynamic diameter of 50 percent by weight of the particles.

As used herein the term "good flowability" refers to a formulation that is easy handled during the manufacturing process and is able of ensuring an accurate and reproducible delivering of the therapeutically effective dose.

Flow characteristics can be evaluated by measuring the Carr's index; a Carr's index of less than 25 is usually taken to indicate good flow characteristics.

As used herein, the expression "good homogeneity" refers to a formulation wherein, upon mixing, the content uniformity of the active ingredient, expressed as relative standard deviation (RSD), is less than 5%.

As used herein, the expression "respirable fraction" refers to an index of the percentage of active particles which would reach the deep lungs in a patient.

The respirable fraction, also termed fine particle fraction, is evaluated using a suitable *in vitro* apparatus such as the Andersen Cascade Impactor or the Mutli Stage Liquid Impinger (MLSI) according to procedures reported in common Pharmacopoeias. It is calculated by the ratio between the respirable dose and the delivered dose. The delivered dose is calculated from the cumulative deposition in the apparatus, while the respirable dose (fine particle dose) is calculated from the deposition on Stages 3 (S3) to filter (AF) corresponding to particles ≤ 4.7 micron.

A respirable fraction higher than 30% is an index of good inhalatory performances.

## DESCRIPTION OF THE DRAWING

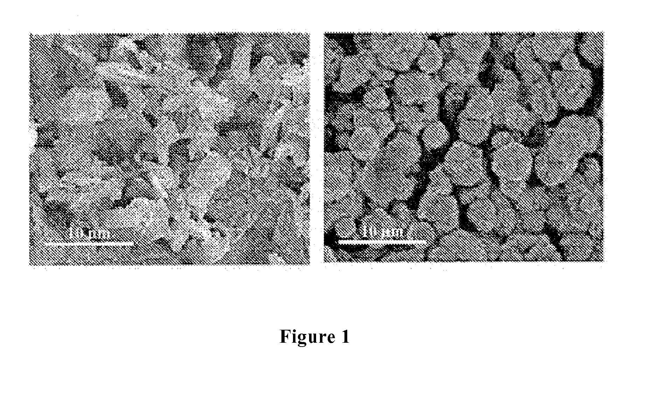

Figure 1 - SEM images of micronised particles carmoterol hydrochloride raw material obtained by conventional milling process (left) and powder particles according to the invention (right). The scale is 10 micron.

## DETAILED DESCRIPTION OF THE INVENTION

The present invention provides powder particles comprising a pharmaceutically acceptable salt of carmoterol characterized by a uniform and regular spherical shape, and by the fact that the salt of carmoterol in said powder particles is present in a substantial amorphous form.

Advantageously the percentage of amorphous salt of carmoterol, expressed as weight % with respect to the total weight of the compound, is equal to or higher than 90%, preferably of at least 95%, even more preferably of at least 98%.

The degree of amorphicity of the salt of carmoterol may be determined using X-ray powder diffraction or other techniques known to the skilled person such as differential scanning calorimetry (DSC) or microcalorimetry.

Preferably carmoterol is used in the form of hydrochloride salt.

The powder particles of the invention exhibit lower adhesion forces than those of the same active substance obtained by conventional micronisation process such as milling.

Therefore said particles, when formulated as interactive ordered mixture with larger carrier particles for administration by inhalation with a dry powder inhaler (DPI) device, could easily and homogeneously disperse in the formulation giving rise to a good uniformity of distribution of the particles, and hence, an adequate accuracy of the metered dose, and, at the same time, to good aerosol performances in terms of percentage of respirable fraction.

It has also been found that particles having a uniform and regular spherical shape exhibit more homogeneous forces of adhesion along the whole powder which are in turn associated with the improved DPI performances.

The shape factor is used to characterize the shape of the particle.

Accordingly, the powder particles of the invention are characterised by a "shape factor" SF comprised between 0.8 and 1.0, preferably from 0.85-and 0.95 as determined according to the following equation reported in Kumar S et al Curr Appl. Phys. Influence of metal powder shape on drag coefficient in a spray jet, 2008, in press doi:10.1016/j.cap. 2008.06.005.

$$SF = 1/RN$$

wherein:

RN indicates the roundness of the particle and is calculated by applying the following formula:

$$RN = p^2/4\pi A$$

wherein p and A are the mean perimeter and area values, respectively, of ten spherical particles as measured from Scanning electron microscopy (SEM) images.

Alternatively, the mean perimeter and area may be measured by an optical microscope.

In Kumar S et al it is reported that the shape factors (SF) of circle is 1.00. It is also reported that deviation from unity leads to irregularity of the particle, but particles with a SF value higher than 0.8 can be considered having a spherical shape.

Scanning electron microscopy (SEM) or optical microscopy may also be used to qualitatively appreciate the characteristics of the powder particles of the invention such as particles shape and their surface morphology.

Another alternative parameter for characterising the shape of the powder particles of the invention is the aerodynamic shape factor.

Therefore, advantageously, the powder particles of the invention are characterised by an "aerodynamic shape factor" $\chi$ comprised between 0.3-1.8 as determined according to the following equation:

$$\chi = \frac{\rho}{\rho_0} \cdot \left(\frac{d_v}{d_{ae}}\right)^2$$

wherein:

d$_{ae}$ is the aerodynamic diameter and is defined as the diameter of a sphere of unit density attaining at low Reynolds numbers in still air the same final settling velocity as the actual particle under consideration;
d$_v$ is the volume-equivalent diameter and is defined as the diameter of a sphere of bulk density having the same volume or mass as the particle under concern, $\rho$ is the particle density and $\rho_0$ is unit density.

Preferably said powder particles have a force of adhesion F$_{max}$ comprised between 12 and 30 nN, preferably between 15 and 28 nN, even more preferably between 18 and 25 nN, upon determination by atomic force microscopy according to the experimental procedure reported in Example 2.

Advantageously the associated work of adhesion W$_{ad}$ shall be comprised between 500 and 2500 $\mu$J, preferably between 600 and 2000 $\mu$J, more preferably between 800 and 1500 $\mu$J.

As a substrate, alpha-lactose particles having the surface characteristics reported in EP 1196146 and obtained as therein described is preferably used. However, other alpha-lactose particles with a smooth surface such as those described in EP0464171 may be advantageously used.

In virtue of said properties, the powder particles of the invention exhibit improved dispersion when formulated as interactive ordered mixtures.

Therefore the powder particles of the invention do not give rise to the formation of stable agglomerates and can be easily dispersed when they are mixed with the coarse carrier particles.

The agglomerates of the active ingredient in the formulations may be detected, for instance, by a Near Infrared Spectrophotometer provided with a microscope, according to methods known to the skilled person.

Once formulated as interactive ordered mixtures, the powder particles of the invention give rise, upon aerosolization, to a good respirable fraction.

Advantageously the powder particles of the invention have a particle size distribution lower than 10 micron, and more advantageously at least 90% of the particles have a diameter equal to or lower than 8 micron as determined by measuring the characteristic equivalent sphere diameter, known as volume diameter, by laser diffraction as described above, preferably using a Malvern or an equivalent apparatus.

Preferably no more than 10% of the powder particles have a volume diameter [d(v,0.1)] lower than 0.8 micron, no more than 50% of have a volume diameter [d(v,0.5)] lower than 1.5 micron, and at least 90% have a volume diameter equal to or lower than 7 micron, preferably equal to or lower than 6 micron.

When measured with a conventional helium picnometer, the real density of the powder particles of the invention is comprised between 1.20 and 1.40 g/cm$^3$, preferably between 1.25 an and 1.30 g/cm$^3$.

The carmoterol salt in the powder particles of the invention is preferably in a substantially pure form (e.g. 95% w/w, preferably 98% or 99% w/w or greater).

Advantageously the powder particles of the invention contain low levels of residual solvent, for example less than 6% w/w, preferably less than 5.0% w/w, most preferably less than 3.0% w/w.

Residual solvents may be determined according to methods well known to the skilled person.

The powder particles of the invention may optionally comprise, incorporated in each unagglomerated individual particle, a further active ingredient, preferably one currently used in the treatment of respiratory disorders such as a corticosteroid.

While the carmoterol salt in the powder particles of the invention is present in a substantial amorphous form, said further optional active ingredient may be present in a crystalline or amorphous form.

The weight ratio between the carmoterol salt and the further active ingredient may be selected from a broad range.

Preferably, the powder particles of the invention further comprise budesonide and when present, the weight ratio of the carmoterol salt to budesonide may be advantageously comprised between 1:10 and 1:400, preferably between 1:50 and 1:320, more preferably between 1:90 and 1:160. In one embodiment of the invention the weight ratio is about 1:100.

The powder particles of the invention may also comprise, incorporated in each unagglomerated individual particle, excipient particles, preferably constituted of lactose, more preferably of alpha-lactose, even more preferably of alpha-lactose monohydrate.

Also in this case, the weight ratio between the carmoterol salt and the excipient may be selected from a broad range, for example from 1:99 to 90:10, preferably from 2:98 to 75:25, more preferably from 10:90 to 70:30, even more preferably from 40:60 to 60:40.

The powder particles of the invention may advantageously be prepared by spray-drying.

Advantageously said method comprising the following steps:

  i. dissolving the salt of carmoterol in an aqueous solution,
  ii. feeding the resulting aqueous solution into the drying chamber of a spray-drier through an atomizing device to form droplets;
  iii. introducing a stream of pre-heated drying gas into the drying chamber to form powder particles;
  iv. further drying and separating the powder particles from the moist gas.

Advantageously, the carmoterol salt is dissolved at a concentration comprised between 0.5 and 10%, preferably from 1 and 5% w/v.

Optionally in step i) a further active ingredient and/or a pharmaceutically acceptable excipient may be present in solution or suspension in a suitable weight ratio.

When a water insoluble active ingredient such budesonide is present, preferably a hydroalcoholic mixture is used, wherein the ratio water: ethanol is 95:5 v/v.

Advantageously, the temperature of the inlet drying gas (inlet temperature) may be maintained between 90 and 200°C, preferably from 100 and 150°C, preferably between 110 and 130°C.

In a particular embodiment, for lab batches, it has also been found that for powder particles constituted of carmoterol hydrochloride only, it would be preferable to keep the inlet temperature equal to or higher than 130°C, preferably comprised between 130 and 150°C to improve the yield of the method.

All the other process parameters of the spray-dryer shall be properly adjusted by the person skilled in the art also in relation with the size of the batch.

Typically, the atomizing device of the spray-dryer may consist of a single or a plurality of nozzles through which the solution is forced by the pump breaking up into droplets.

The nozzles may have different diameters.

In one embodiment of the invention, the atomizing device is a single nozzle and its diameter is 1.0 mm.

However, for the spraying process, other kinds of atomizing device such as a rotary (centrifugal) atomizing device or other suitable devices may be used. The person skilled in the art shall adapt the various process conditions and parameters accordingly.

Although, spray-drying remains the preferred method for preparing the powder particles of the invention, other suitable methods may be used such as those methods based on the use of gases compressed and/or supercritical conditions. An example of said methods is the SEDS™ method described in WO 95/01221.

In another aspect, the present invention relates to a powder formulation for inhalation under the form of interactive ordered mixtures characterised in that it comprises the powder particles according to the invention.

Advantageously, said powder formulation for inhalation may comprise the powder particles according to the invention and coarse particles of a physiologically acceptable excipient, e.g. particles having a MMD higher than 90 micron and preferably the MD comprised between 50 micron and 500 micron, more preferably between 150 and 400 micron, even more preferably between 210 and 355 micron. In another embodiment, the coarse particles have a MD comprised between 90 and 150 micron.

In one of the preferred embodiment, when their MD is comprised between 210 and 355 micron, the coarse excipient particles have preferably a relatively highly fissured surface, that is, on which there are clefts and valleys and other recessed regions, referred to herein collectively as fissures.

The "relatively highly fissured" surface of the coarse excipient particles may be defined in terms of fissure index or rugosity coefficients as disclosed in WO 01/78695 and WO 01/78693 and they can be characterized according to the description therein reported.

Preferably the relevant powder formulation may further comprises a fraction of pharmacologically-inert microparticles having a MMD lower than 35 micron composed of particles of a physiologically acceptable excipient and an additive material selected from the class of the anti-adherents such as the amino acids leucine and isoleucine or of the lubricants such as magnesium stearate; sodium stearyl fumarate stearyl alcohol, stearic acid and sucrose monopalmitate.

More preferably said powder formulation comprises a fraction of said pharmacologically-inert microparticles having a MMD lower than 15 micron, preferably lower than 10 micron, composed of particles of a physiologically acceptable excipient and particles of magnesium stearate according to the teaching of EP 1274406.

In another preferred embodiment of the invention, when their MD is comprised between 90 and 150 micron, the coarse carrier particles have preferably a surface rugosity expressed as the fractal dimension of less than or equal to 1.1, determined according to the teaching of EP 1196146. More preferably the surface of said particles is coated with magnesium stearate.

Magnesium stearate is added to the formulations herein described the aim of improving the respirable fraction of the active substance.

The physiologically acceptable excipient may be constituted of any amorphous or crystalline physiologically acceptable pharmacologically-inert material of animal or vegetal source or combination thereof. Preferred materials are crystalline sugars and for example monosaccharides such as glucose or arabinose, or disaccharides such as maltose, saccharose, dextrose or lactose. Polyalcohols such as mannitol, sorbitol, maltitol, lactitol may also be used. The most preferred material is α-lactose monohydrate.

Examples of commercial lactose are Capsulac™ and Pharmatose™. An example of commercial mannitol is Pearlitol™.

In a preferred embodiment, the fraction of microparticles is composed of 98% by weight of α-lactose monohydrate and 2% by weight of magnesium stearate and the ratio between the fraction of microparticles and the fraction of coarse particles made of α-lactose monohydrate particles is 10:90% by weight, respectively.

The amount of magnesium stearate in the final formulation is advantageously comprised between 0.02% and 1.0% by weight on the total weight of the formulation, preferably between 0.05 and 0.5% by weight, more preferably between 0.1 and 0.4% by weight, even more preferably between 0.2 and 0.3% by weight.

The powder formulation for inhalation comprising the powder particles according to the invention is characterized by a high degree of homogeneity. After the mixing, the content uniformity of the active ingredient, expressed as relative standard deviation (RSD), is less than 5%, preferably equal/less than 3.5%, more preferably equal or less than 1.5%.

Said powder formulation may be administered by inhalation with any type of DPIs known in the art.

DPIs can be divided into two basic types: i) single dose inhalers, for the administration of pre-subdivided single doses of the active compound; ii) multidose dry powder inhalers (MDPIs), either with pre-subdivided single doses or pre-loaded with quantities of active ingredient sufficient for multiple doses. On the basis of the required inspiratory flow rates (1/min) which in turn are strictly depending on their design and mechanical features, DPIs are divided in: i) low-resistance devices (> 90 1/min); ii) medium-resistance devices (about 60 l/min); iii) high-resistance devices (about 30 1/min).

Having regard to its beta$_2$-adrenoceptor stimulating activity, carmoterol is useful in the relaxation of bronchial smooth muscle and the relief of bronchoconstriction.

Therefore the carmoterol-comprising powder particles of the invention may be indicated for the prevention and/or treatment of mild, moderate or severe acute or chronic symptoms or for prophylactic treatment of respiratory diseases such as asthma and chronic obstructive pulmonary disease (COPD). Other respiratory disorders characterized by obstruction of the peripheral airways as a result of inflammation and presence of mucus such as chronic obstructive bronchiolitis and chronic bronchitis may also benefit by their use.

The invention is further illustrated by the following examples.

## EXAMPLES

**Example 1 - Preparation of powder particles according to the invention by spray-drying**

Different batches of powder particles according to the invention are prepared by spray-drying starting from an aqueous solution or a hydroalcoholic solution of carmoterol hydrochloride.

The 1% w/v solutions are loaded into a Mini Spray Dryer B-191 (Buchi, Switzerland) equipped with a pressure atomizing device consisting of a nozzle.

The following process parameters are utilised: nozzle diameter: 1.0 mm; air flow rate: 600 1/h; feed flow rate: 0.195 1/h; inlet temperature: 130°C.

The powder particles in the form of free-flowing powders are collected under the cyclone of the spray-dryer through a rotary valve.

The quali-quantitative composition of the obtained microparticles and their relevant yields are reported in Table 1.

**Table 1 - Quali-quantitative composition of the powder particles obtained by spray-drying and relevant yields**

| Batch | Carmoterol HCl (%, w/w) | Budesonide (%, w/w) | $\alpha$-Lactose (%, w/w) | Ethanol (%, v/v) | Yield (%) |
|---|---|---|---|---|---|
| Batch 1 | 100 | 0 | 0 | 0 | 72.4 |
| Batch 2 | 50 | 0 | 50 | 0 | 71.2 |
| Batch 3 | 1 | 99 | 0 | 5 | 69.8 |

Inspection by scanning electron microscope (SEM) indicates that, independently on the composition, the obtained powder particles exhibit an uniform and regular spherical shape in comparison to micronised carmoterol hydrochloride obtained by a conventional milling process which exists in the form of different irregular flakes and/or sheets (see Figure 1).

The shape factor of the obtained particles, calculated according to the equation reported in the *Detailed Description of the Invention,* turned out to be comprised between 0.87 and 0.95.

For sake of comparison, carmoterol hydrochloride raw material obtained by a conventional milling process exhibit a SF value of 0.51.

The X-ray powder diffraction (XPRD) analysis of batches 1 and 2 shows a characteristic diffused pattern associated with substantial amorphous materials.

In the XPRD pattern of batch 3, only the diffraction peaks of crystalline budesonide were detected as it is the overwhelming part of the particles. Unsurprisingly, the diffused pattern of amorphous carmoterol hydrochloride could not be observed due to its very low amount.

The batches of powder particles have also been characterised in terms of particle size distribution and real density.

The particle size has been determined by laser diffraction using a Mastersize X apparatus. The parameters taken into consideration are the volume diameters (VD) in micron of 10%, 50% and 90% of the particles expressed as d(v,0.1), d(v, 0.5) and d(v, 0.9), respectively, which correspond to the mass diameter assuming a size independent density for the particles. The mean values of three samples are reported in Table 2. The standard deviation (S.D.) turns out to be less than $\pm$ 0.2.

The real density has been determined using a helium picnometer apparatus. The mean values of three samples and the S.D. are reported in Table 3.

**Table 2 - Particle size distribution**

|  | Batch 1 | Batch 2 | Batch 3 |
|---|---|---|---|
| Particle size ($\mu$m) |  |  |  |
| d(v, 0.1) | 0.57 | 1.11 | 0.79 |
| d(v, 0.5) | 1.65 | 1.94 | 2.98 |
| d(v, 0.9) | 3.90 | 3.40 | 6.81 |

**Table 3 - Real density**

| Batch | Real density (g/cm$^3$) |
|---|---|
| Batch 1 | 1.246 $\pm$ 0.018 |
| Batch 2 | 1.390 $\pm$ 0.026 |
| Batch 3 | 1.243 $\pm$ 0.016 |

**Example 2 - Determination of the force of adhesion of the powder particles of the invention**

The force of adhesion of the batches of powder particles obtained in Example 1 has been determined according to the procedure reported hereafter in comparison to those to those of micronised carmoterol hydrochloride and budesonide raw materials (r.m.) obtained by a conventional milling process.

As a substrate, alpha-lactose particles with a smooth surface having the characteristics reported in EP 1196146 and obtained as therein described are used.

Due to their smooth surface, said particles allow to decrease the variability of the data, and hence, to obtain reliable measurements.

An Atomic Force Microscope (AFM) as a scanning probe microscope is used to image lactose surface on an nm or even sub-nm level and to measure surface forces between the powder particles of the invention and particles of smoothed lactose.

The surface topography of the smoothed lactose surfaces is investigated using the AFM in a conventional non contact imaging mode with a non-conductive silicon nitride cantilever (spring constant = 0.58 N/m, Veeco Instruments Ltd., Cambridge, UK), at a scan rate of 20.03 $\mu$m/s with an Explorer AFM (Thermomicroscopes, UK). The root-mean squared surface roughness (rms) of each sample is calculated from the AFM height data over a 10 $\mu$m x 10 $\mu$m area using equation

$$R_{rms} = \sqrt{\frac{1}{n}\sum_{i-1}^{n} y_i^2}$$

where n is the number of points in the topography profile and yi is the distance of asperities (i) from the centre line.

The AFM images of the lactose carrier display similar characteristics to those highlighted by the SEM pictures. Using the $R_{rms}$ the roughness of the surface is significantly low (227.1 $\pm$ 45.2 nm) for the smooth lactose used in this work to measure the force of adhesion as described below.

A way to get information on the surface forces which are acting in a system and to illustrate the functioning of an AFM, is to use the "Force-Mode". This is done by moving the tip towards and away from the surface. Recording photodetector signal as a function of z-piezo elongation yields a curve which can be interpreted as a force-vs.-distance-curve. In this work this technique has been employed to measure the drug-carrier adhesion force modifying the original cantilever.

The interaction of the micron sized carmoterol particles with lactose carrier surfaces is conducted using the Explorer AFM (Thermomicroscopes, UK). A multistage micromanipulation method for the attachment of micrometer-sized drug particles to tipless cantilevers was adapted from the method described by Preuss and Butt (Preuss M, Butt H-J. 1998, Langmuir 14:3164-3174). Essentially, colloid probes are prepared by mounting an individual drug particle (approximate diameter 3-5 $\mu$m) onto a V-shaped tiples silicon nitride cantilever (spring constant k = 0.58 N/m, Veeco Instruments Ltd., Cambridge, UK) using a quick-setting epoxy resin together with the custom-built microscope.

Force separation measurements are collected using layered imaging mode to produce multiple force distance curves (n = 250) between each drug probe at three surface sites over a 10 $\mu$m x 10 $\mu$m area. As the variability in probe contact radius geometry was unknown each study is performed at least in triplicate (n = 3-4 probes for each measurement).

The displacement curves for each of the 250 contact points in the 10 $\mu$m x 10 $\mu$m sector are plotted using a custom built MatLab program (The Mathworks Inc. USA). This data is converted into force displacement curves using the region of constant compliance for each curve and spring constant according to Hookes law (described in detail: R. Price, P. M. Young, S. Edge, and J. N. Staniforth. The influence of relative humidity on particulate interactions in carrier-based dry powder inhaler formulations, Int J Pharm 2002, 246 (1-2), 47-59).

The force of adhesion (the apex of the adhesion peak) and the work of adhesion (integrated area of the adhesion peak) is calculated for each graph. This data is plotted as a histogram and the mean force of adhesion and work of adhesion determined for the sum of the 250 contact points. Outliers are defined as data points out side the 5-95% limits of the normal distributions obtained and these are removed automatically by the batch conversion software.

The obtained mean values $\pm$ S.D. of force of adhesion $F_{max}$ between the different batches of microparticles and the smooth lactose carrier as well as the corresponding work of adhesion $W_{ad}$ are reported in Table 4.

For sake of comparison, the values of micronised carmoterol hydrochloride and budesonide raw materials (r.m.) obtained by a conventional milling process are reported.

**Table 4 - Force of adhesion ($F_{max}$) and corresponding work of adhesion ($W_{ad}$)**

| Batch | $F_{max}$ (nN) | $W_{ad}$ ($\mu$J) |
|---|---|---|
| Batch 1 | 22.46 $\pm$ 3.17 | 957.28 $\pm$ 290.41 |
| Batch 2 | 12.89 $\pm$ 3.34 | 711.87 $\pm$ 221.31 |
| Batch 3 | 22.25 $\pm$ 3.13 | 1430.36 $\pm$ 328.37 |
| Carmoterol HCl r.m. | 11.03 $\pm$ 1.27 (20%) | 413.15 $\pm$ 131.85 |
|  | 42.68 $\pm$ 4.63 (80%) | 2115.69 $\pm$ 344.31 |
| Budesonide r.m | 57.40 $\pm$ 6.56 | 4266.79 $\pm$ 430.78 |

It can be appreciated that the batch of budesonide raw material shows a force of adhesion much higher than that of the batches of powder particles obtained in Example 1.

Carmoterol hydrochloride raw material shows different values corresponding to at least two populations of particles having different morphology, indicating that said active ingredient may also face problems of dispersion reproducibility in addition to poor dispersion.

**Example 3 - "Interactive ordered mixture" formulation comprising the powder particles batch 1 of Example 1**

The powder particles batch 1 as obtained in Example 1 has been added to a carrier prepared according to the teaching of EP 1274406 and reported hereafter.

a) Preparation of the fraction of the pharmacologically-inert microparticles. $\alpha$-lactose monohydrate SpheroLac® 100 with a starting mass diameter of 50 to 400 micron (MMD of about 170 micron) and magnesium stearate particles in the ratio 98:2 percent by weight are co-milled in a jet mill apparatus until the MMD of the whole mixture is less than 15 micron.

b) Addition of the fraction of microparticles to the fraction of coarse particles. 90 percent by weight of $\alpha$-lactose monohydrate CapsuLac® (212-355 micron) is placed in a 240 ml stainless steel container, then 10 percent by weight of the fraction of pharmacologically-inert microparticles is added. The blend is mixed in a Turbula mixer for 2 hours at 42 r.p.m. to obtain the carrier.

c) Addition of the powder particles of the invention to the carrier.

The batch 1 is added to the carrier in a suitable amount in order to obtain a ratio of 2 $\mu$g of carmoterol hydrochloride to 10 mg of final formulation. The resulting blend is mixed in a Turbula mixer for 30 min at 32 r.p.m.

For sake of comparison, an analogous formulation is prepared by adding micronised particles of carmoterol hydrochloride obtained by a conventional milling process.

Batch 1 uniformly disperses into the carrier and after one hour of mixing no agglomerates are observed.

On the contrary, agglomerates where still present in the comparative formulation after much longer period, i.e. ten hours of mixing.

**Example 4 - Characterisation of the powder formulation of Example 3**

The powder formulation of Example 3 has been characterised in terms of the uniformity of distribution of the active ingredient and aerosol performances after loading it in the multidose dry powder inhaler Pulvinal™.

The uniformity of distribution of the active ingredient is evaluated by withdrawing six samples from different parts of the blend and evaluated by HPLC.

The evaluation of the aerosol performance is carried out using the Andersen Cascade Impactor (Apparatus D) according to the conditions reported in the European Pharmacopoeia 6th Ed 2008, par 2.9.18, pages 293-295.

After aerosolization of 10 doses, the ACI apparatus is disassembled and the amounts of drug deposited in the stages were recovered by washing with a solvent mixture and then quantified by High-Performance Liquid Chromatography (HPLC). The following parameters, are calculated: *i)* the delivered dose which is the amount of drug delivered from the device recovered in the impactor; *ii)* the fine particle dose (FPD) which is the amount of delivered dose recovered in the S3-AF stages having a particle size equal to or lower than 4.7 micron; *iii)* the fine particle fraction (FPF) which is the percentage of the fine particle dose; iv) the MMAD.

The results in terms of uniformity of distribution, FPF and MMAD (mean value $\pm$ S.D) are reported in Table 5.

**Table 5 - Uniformity of distribution of the active ingredient and aerosol performances**

| Uniformity of distribution of the active ingredient (%, RSD) | $97.5 \pm 2.6$ |
|---|---|
| FPF (%) | $51.5 \pm 0.31$ |
| MMAD ($\mu$m) | $3.74 \pm 0.06$ |

The formulation prepared using the powder particles of the invention shows an excellent good uniformity of distribution of the active ingredient as demonstrated by the low RSD. The aerosol performances of the formulation are very good with more than 50% of FPF.

Moreover, the powder formulation turns out to be physically and chemically stable when stored for at least six months under long-term stability conditions (25°C, 60% relative humidity).

**Claims**

1.  Powder particles for administration through a dry powder inhaler comprising a pharmaceutically acceptable salt of 8-hydroxy-5-[(1R)-1-hydroxy-2-[[(1R)-2-(4-methoxyphenyl)-1-methylethyl]amino]ethyl-2(1H)-quinolinone (carmoterol) **characterized by** a shape factor SF comprised between 0.8 and 1.0 and by the fact that the salt of carmoterol in said powder particles is present in a substantial amorphous form.

2.  The powder particles according to claim 1 wherein the salt of carmoterol is the hydrochloride salt.

3.  The powder particles of claim 1 or 2 wherein the shape factor SF is comprised 0.85 and 0.95.

4.  The powder particles according to any one of the preceding claims wherein the salt of carmoterol is present with a degree of amorphicity equal to or higher than 90%.

5.  The powder particles according to any one of the preceding claim s further **characterized by** a force of adhesion $F_{max}$ comprised between 12 and 30 nN.

6.  The powder particles according to claim 5 wherein the force of adhesion $F_{max}$ is comprised between 15 and 28 nN.

7.  The powder particles according to claim 6 wherein the force of adhesion $F_{max}$ is comprised between 18 and 25 nN.

8.  The powder particles according to any one of the preceding claims having a particle size distribution lower than 10 micron.

9.  The powder particles according to claim 8 wherein at least 90% of the particles have a diameter equal to or lower than 8 micron.

10. The powder particles according to claim 9 wherein:

    i) no more than 10% of the particles have a volume diameter lower than 0.8 micron;
    ii) no more than 50% of particles have a volume diameter lower than 1.5 micron; and
    iii) at least 90% of the particles have a volume diameter equal to or lower than 7 micron.

11. The powder particles according to any one of the preceding claims further comprising a further active ingredient selected from the class of corticosteroids.

12. The powder particles according to claim 11 wherein the corticosteroid is budesonide.

13. The powder particles according to any of the preceding claims further comprising a pharmaceutically acceptable excipient.

14. The powder particles according to claim 13 wherein the physiologically acceptable excipient is alpha-lactose monohydrate.

**15.** A process for the preparation of the powder particles of any one of the preceding claims which comprises the following steps:

  i) dissolving the salt of carmoterol in an aqueous solution;
  ii) feeding the resulting aqueous solution into the drying chamber of a spray-drier through an atomizing device to form droplets;
  iii) introducing a stream of pre-heated drying gas into the drying chamber to form powder particles;
  iv) further drying and separating the powder particles from the moist gas.

**16.** A powder formulation to be administered by inhalation using a dry powder inhaler device comprising the powder particles of any one of claims 1 to 14.

**17.** The powder formulation according to claim 16 further comprising a physiologically acceptable excipient having a mass median diameter (MMD) higher than 90 micron.

**18.** The powder formulation according to claim 17 further comprising a fraction of pharmacologically-inert microparticles having a MMD lower than 15 micron constituted of a mixture of particles of a physiologically acceptable excipient and magnesium stearate.

**19.** The powder formulation according to claims 17 or 18 wherein the physiologically acceptable material is alpha-lactose monohydrate.

**20.** A dry powder inhaler comprising a powder formulation according to any one of claims 16 to 19.

**21.** Use of powder particles according to any one of claims 1 to 14 for the treatment of a pulmonary disease.

**Figure 1**

| | Europäisches Patentamt European Patent Office Office européen des brevets | **EUROPEAN SEARCH REPORT** | **Application Number** EP 08 16 9993 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 944 018 A (CHIESI FARMA SPA [IT]) 16 July 2008 (2008-07-16) | 1-21 | INV. A61K9/14 |
| Y | * paragraphs [0031] - [0043], [0048] * ----- | 15 | A61K9/72 A61K31/4704 |
| X | WO 2005/089717 A (CHIESI FARMA SPA [IT]; BILZI ROBERTO [IT]; ARMANNI ANGELA [IT]; RASTEL) 29 September 2005 (2005-09-29) * page 26; example 1 * ----- | 1-14, 16-21 | A61K47/26 |
| X | US 2003/180227 A1 (STANIFORTH JOHN NICHOLAS [IT] ET AL) 25 September 2003 (2003-09-25) * paragraph [0039]; claim 3; examples * ----- | 1-14, 16-21 | |
| Y | WO 96/32095 A (ASTRA AB [SE]; JAKUPOVIC EDIB [SE]; TROFAST JAN [SE]) 17 October 1996 (1996-10-17) * examples * ----- | 15 | |
| | | | **TECHNICAL FIELDS SEARCHED (IPC)** A61K |

The present search report has been drawn up for all claims

| Place of search | Date of completion of the search | Examiner |
|---|---|---|
| Munich | 6 April 2009 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 16 9993

This annex lists the patent family members relating to the patent documents cited in the above-mentioned European search report.
The members are as contained in the European Patent Office EDP file on
The European Patent Office is in no way liable for these particulars which are merely given for the purpose of information.

06-04-2009

| Patent document cited in search report | | Publication date | Patent family member(s) | | Publication date |
|---|---|---|---|---|---|
| EP 1944018 | A | 16-07-2008 | WO | 2008084312 A2 | 17-07-2008 |
| WO 2005089717 | A | 29-09-2005 | AR | 049010 A1 | 21-06-2006 |
| | | | AU | 2005224008 A1 | 29-09-2005 |
| | | | BR | PI0508170 A | 07-08-2007 |
| | | | CA | 2560226 A1 | 29-09-2005 |
| | | | CN | 1942172 A | 04-04-2007 |
| | | | KR | 20060130216 A | 18-12-2006 |
| | | | US | 2007202053 A1 | 30-08-2007 |
| US 2003180227 | A1 | 25-09-2003 | AT | 348603 T | 15-01-2007 |
| | | | AT | 377416 T | 15-11-2007 |
| | | | AT | 339195 T | 15-10-2006 |
| | | | AU | 784719 B2 | 01-06-2006 |
| | | | AU | 4858101 A | 30-10-2001 |
| | | | AU | 4859501 A | 30-10-2001 |
| | | | AU | 5834301 A | 30-10-2001 |
| | | | BR | 0110139 A | 31-12-2002 |
| | | | BR | 0110141 A | 28-01-2003 |
| | | | BR | 0110301 A | 30-12-2003 |
| | | | CA | 2405767 A1 | 25-10-2001 |
| | | | CA | 2406119 A1 | 25-10-2001 |
| | | | CA | 2406201 A1 | 25-10-2001 |
| | | | CN | 1424909 A | 18-06-2003 |
| | | | CZ | 20023437 A3 | 12-02-2003 |
| | | | DE | 60123031 T2 | 08-03-2007 |
| | | | DE | 60125344 T2 | 19-07-2007 |
| | | | DE | 60131265 T2 | 06-03-2008 |
| | | | DK | 1276472 T3 | 16-04-2007 |
| | | | DK | 1274406 T3 | 22-01-2007 |
| | | | EE | 200200593 A | 15-04-2004 |
| | | | WO | 0178693 A2 | 25-10-2001 |
| | | | EP | 1276472 A2 | 22-01-2003 |
| | | | EP | 1276473 A2 | 22-01-2003 |
| | | | EP | 1274406 A2 | 15-01-2003 |
| | | | EP | 1719505 A2 | 08-11-2006 |
| | | | EP | 1829533 A2 | 05-09-2007 |
| | | | ES | 2275669 T3 | 16-06-2007 |
| | | | ES | 2292576 T3 | 16-03-2008 |
| | | | ES | 2272473 T3 | 01-05-2007 |
| | | | WO | 0178694 A2 | 25-10-2001 |
| | | | WO | 0178695 A2 | 25-10-2001 |
| | | | GB | 2363987 A | 16-01-2002 |
| | | | GB | 2363988 A | 16-01-2002 |
| | | | HU | 0300490 A2 | 28-07-2003 |
| | | | HU | 0300499 A2 | 28-07-2003 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**                    EP 08 16 9993

This annex lists the patent family members relating to the patent documents cited in the above-mentioned European search report.
The members are as contained in the European Patent Office EDP file on
The European Patent Office is in no way liable for these particulars which are merely given for the purpose of information.

06-04-2009

| Patent document cited in search report | Publication date | Patent family member(s) | | Publication date |
|---|---|---|---|---|
| US 2003180227 A1 | | HU | 0300593 A2 | 29-09-2003 |
| | | JP | 2003530425 T | 14-10-2003 |
| | | MA | 26892 A1 | 20-12-2004 |
| | | MX | PA02010212 A | 29-06-2004 |
| | | MX | PA02010213 A | 01-07-2005 |
| | | MX | PA02010218 A | 23-05-2003 |
| | | NO | 20024971 A | 17-12-2002 |
| | | NO | 20024973 A | 16-12-2002 |
| | | NO | 20024980 A | 17-12-2002 |
| | | NZ | 521887 A | 25-06-2004 |
| | | PL | 358640 A1 | 09-08-2004 |
| | | PL | 358875 A1 | 23-08-2004 |
| | | PL | 359289 A1 | 23-08-2004 |
| WO 9632095 A | 17-10-1996 | AR | 001624 A1 | 26-11-1997 |
| | | AT | 230257 T | 15-01-2003 |
| | | AU | 694863 B2 | 30-07-1998 |
| | | AU | 5352496 A | 30-10-1996 |
| | | CA | 2217062 A1 | 17-10-1996 |
| | | CN | 1186428 A | 01-07-1998 |
| | | DE | 69625589 D1 | 06-02-2003 |
| | | DE | 69625589 T2 | 25-09-2003 |
| | | DK | 820276 T3 | 17-03-2003 |
| | | EP | 0820276 A1 | 28-01-1998 |
| | | ES | 2188750 T3 | 01-07-2003 |
| | | IL | 117841 A | 04-01-2004 |
| | | IN | 185119 A1 | 18-11-2000 |
| | | JP | 11503448 T | 26-03-1999 |
| | | NO | 974557 A | 02-10-1997 |
| | | NZ | 305515 A | 29-03-1999 |
| | | PT | 820276 E | 30-04-2003 |
| | | TW | 492877 B | 01-07-2002 |
| | | US | 6221398 B1 | 24-04-2001 |
| | | ZA | 9602596 A | 14-10-1996 |

EPO FORM P0459

For more details about this annex : see Official Journal of the European Patent Office, No. 12/82

**REFERENCES CITED IN THE DESCRIPTION**

**Patent documents cited in the description**

- WO 2005089717 A
- WO 2008084312 A
- EP 1196146 A
- EP 0464171 A
- WO 9501221 A
- WO 0178695 A
- WO 0178693 A
- EP 1274406 A

**Non-patent literature cited in the description**

- **Kumar S et al.** Influence of metal powder shape on drag coefficient in a spray jet. *Curr Appl. Phys,* 2008
- **Preuss M ; Butt H-J.** *Langmuir,* 1998, vol. 14, 3164-3174
- **R. Price ; P. M. Young ; S. Edge ; J. N. Staniforth.** The influence of relative humidity on particulate interactions in carrier-based dry powder inhaler formulations. *Int J Pharm,* 2002, vol. 246 (1-2), 47-59
- European Pharmacopoeia. 2008, 293-295